# EUROPEAN PATENT APPLICATION

(11) **EP 2 446 883 A1**
(43) Date of publication of application: **02.05.2012**
(21) Application number: 10189534.0
(22) Date of filing: 30.10.2010
(51) Int. Cl.: A61K 31/00, A61K 31/4015, G01N 33/50, A61P 29/00, A61P 9/00, A61P 25/28, A61P 35/00, A61P 37/06, A61P 9/10

(54) **Treatment of inflammation**

(71) Applicant: University College Cork-National University of Ireland, Cork, Cork (IE)
(72) Inventor: Carmody, Ruadhri, Dr., Cork (IE)
(74) Representative: Purdy, Hugh Barry

(57) **Abstract**

A method for the treatment of inflammation in an individual is described, for example a causal therapy of a disease or condition for which an inflammatory state is an underlying cause of the disease, or a treatment of inflammation as a symptom of a disease state, for example inflammation caused by cancer, cardiovascular disease, an infection or trauma, by treating the individual with an agent capable of attenuating the activity of Ubiquitin Serine Protease 7 (USP7) protein, in particular a USP7 inhibitor chemical.

SEQUENCE LISTING

<110> UNIVERSITY COLLEGE CORK, NATIONAL UNIVERSITY OF IRELAND,
CORK

<120> TREATMENT OF INFLAMMATION

<130> P10579EP00

<140> EP10189534.0
<141> 2010-10-30

<160> 42

<170> PatentIn version 3.5

<210> 1
<211> 21
<212> RNA
<213> Artificial sequence

<220>
<223> siRNA sense strand


<220>
<221> misc_feature
<222> (1)..(21)
<223> siRNA molecule sense strand

<400> 1
cccaaauuau uccgcggcaa a
21


<210> 2
<211> 21
<212> RNA
<213> Homo sapiens


<220>
<221> misc_feature
<222> (1)..(21)
<223> siRNA Target sequence in USP7 mRNA

<400> 2
cccaaauuau uccgcggcaa a
21


<210> 3
<211> 21
<212> RNA

<213>  Artificial sequence

<220>
<223>  siRNA molecule


<220>
<221>  misc_feature
<222>  (1)..(21)
<223>  siRNA specific to nucleotides 761-778 of human USP7 mRNA

<400>  3
cccaaauuau uccgcggcaa a
21


<210>  4
<211>  21
<212>  DNA
<213>  Homo sapiens


<220>
<221>  misc_feature
<222>  (1)..(21)
<223>  siRNA target sequence in USP7 mRNA

<400>  4
aagcgtccct ttagcattat t
21


<210>  5
<211>  21
<212>  RNA
<213>  Artificial sequence

<220>
<223>  siRNA molecule


<220>
<221>  misc_feature
<222>  (1)..(21)
<223>  siRNA antisense strand

<400>  5
uuugccgcgg aauaauuugg g
21


<210>  6
<211>  21
<212>  RNA

<213>  Artificial sequence

<220>
<223>  siRNA molecule


<220>
<221>  misc_feature
<222>  (1)..(21)
<223>  siRNA specific to mouse USP7 mRNA

<400>  6
cagguagaca uagauaagga a
21


<210>  7
<211>  21
<212>  RNA
<213>  Artificial sequence

<220>
<223>  siRNA molecule


<220>
<221>  misc_feature
<222>  (1)..(21)
<223>  siRNA specific to mouse USP7 mRNA

<400>  7
aaagaucaug auguaauguu a
21


<210>  8
<211>  21
<212>  RNA
<213>  Artificial sequence

<220>
<223>  siRNA molecule


<220>
<221>  misc_feature
<222>  (1)..(21)
<223>  siRNA specific to mouse USP7 mRNA

<400>  8
caggauuuau ccaggauacu a
21

<210>  9
<211>  21
<212>  RNA
<213>  Artificial sequence

<220>
<223>  siRNA molecule


<220>
<221>  misc_feature
<222>  (1)..(21)
<223>  siRNA specific to mouse USP7 mRNA

<400>  9
cugauggaug gugacaucau a
21


<210>  10
<211>  21
<212>  DNA
<213>  Artificial sequence

<220>
<223>  siRNA molecule


<220>
<221>  misc_feature
<222>  (1)..(21)
<223>  siRNA (sense) specific to mouse USP7 mRNA

<400>  10
gguagacaua gauaaggaat t
21


<210>  11
<211>  21
<212>  DNA
<213>  Artificial sequence

<220>
<223>  siRNA molecule


<220>
<221>  misc_feature
<222>  (1)..(21)
<223>  siRNA (antisense) specific to mouse USP7 mRNA

<400>  11

uuccuuaucu augucuacct g
21

<210>  12
<211>  27
<212>  DNA
<213>  Artificial sequence

<220>
<223>  Primer

<220>
<221>  misc_feature
<222>  (1)..(27)
<223>  IL-6 Forward Primer

<400>  12
cgtttatgat tctttcgatg ctaaacg
27

<210>  13
<211>  22
<212>  DNA
<213>  Artificial sequence

<220>
<223>  Primer

<220>
<221>  misc_feature
<222>  (1)..(22)
<223>  IL-6  Reverse Primer

<400>  13
gtggggtcca gagcagaatg ag
22

<210>  14
<211>  24
<212>  DNA
<213>  Artificial sequence

<220>
<223>  Primer

<220>
<221>  misc_feature
<222>  (1)..(24)

<223>  TNF-alpha forward primer

<400>  14
gaggctccgt ggaaaactca cttg
24


<210>  15
<211>  24
<212>  DNA
<213>  Artificial sequence

<220>
<223>  Primer


<220>
<221>  misc_feature
<222>  (1)..(24)
<223>  TNF-alpha reverse primer

<400>  15
gcagagcagc ttgagagttg ggaa
24


<210>  16
<211>  19
<212>  DNA
<213>  Artificial sequence

<220>
<223>  Primer


<220>
<221>  misc_feature
<222>  (1)..(19)
<223>  IkappaBalpha forward primer

<400>  16
tcaaatcgat cgtgggaaa
19


<210>  17
<211>  18
<212>  DNA
<213>  Artificial sequence

<220>
<223>  Primer

<220>
<221>  misc_feature
<222>  (1)..(18)
<223>  I kappa B alpha reverse primer

<400>  17
ggactgctgt gggctctg
18


<210>  18
<211>  21
<212>  DNA
<213>  Artificial sequence

<220>
<223>  Primer


<220>
<221>  misc_feature
<222>  (1)..(21)
<223>  Beta Actin forward primer

<400>  18
tcgatatcca cgtgacatcc a
21


<210>  19
<211>  21
<212>  DNA
<213>  Artificial sequence

<220>
<223>  Primer


<220>
<221>  misc_feature
<222>  (1)..(21)
<223>  Beta Actin reverse primer

<400>  19
gcagcatttt tttaccccct c
21


<210>  20
<211>  21
<212>  DNA
<213>  Mus musculus

<400>  20

caggtagaca tagataagga a
21

<210> 21
<211> 21
<212> DNA
<213> Artificial sequence

<220>
<223> Primer

<220>
<221> misc_feature
<222> (1)..(21)
<223> IL-6 forward primer

<400> 21
tgatggatgc taccaaactg g
21

<210> 22
<211> 24
<212> DNA
<213> Artificial sequence

<220>
<223> Primer

<220>
<221> misc_feature
<222> (1)..(24)
<223> Il-6 reverse primer

<400> 22
ttcatgtact ccaggtagct atgg
24

<210> 23
<211> 21
<212> DNA
<213> Artificial sequence

<220>
<223> Primer

<220>
<221> misc_feature
<222> (1)..(21)

<223>  TNF-alpha forward primer

<400>  23
tcttctcatt cctgcttgtg g
21


<210>  24
<211>  20
<212>  DNA
<213>  Artificial sequence

<220>
<223>  Primer


<220>
<221>  misc_feature
<222>  (1)..(20)
<223>  TNF-alpha reverse primer

<400>  24
ggtctgggcc atagaactga
20


<210>  25
<211>  20
<212>  DNA
<213>  Artificial sequence

<220>
<223>  Primer


<220>
<221>  misc_feature
<222>  (1)..(20)
<223>  IkBalpha forward primer

<400>  25
acgagcaaat ggtgaaggag
20


<210>  26
<211>  19
<212>  DNA
<213>  Artificial sequence

<220>
<223>  Primer

<220>
<221>  misc_feature
<222>  (1)..(19)
<223>  IkBalpha reverse primer

<400>  26
atgattgcca agtgcagga
19


<210>  27
<211>  24
<212>  DNA
<213>  Artificial sequence

<220>
<223>  Primer


<220>
<221>  misc_feature
<222>  (1)..(24)
<223>  18S rRNA forward primer

<400>  27
aaatcagtta tggttccttt ggtc
24


<210>  28
<211>  26
<212>  DNA
<213>  Artificial sequence

<220>
<223>  Primer


<220>
<221>  misc_feature
<222>  (1)..(26)
<223>  18S rRNA reverse primer

<400>  28
gctctagaat taccacagtt atccaa
26


<210>  29
<211>  20
<212>  DNA
<213>  Artificial sequence

<220>

<223>  Primer


<220>
<221>  misc_feature
<222>  (1)..(20)
<223>  IL-10 forward primer

<400>  29
cagagccaca tgctcctaga
20


<210>  30
<211>  20
<212>  DNA
<213>  Artificial sequence

<220>
<223>  Primer


<220>
<221>  misc_feature
<222>  (1)..(20)
<223>  IL-10 reverse primer

<400>  30
gtccagctgg tcctttgttt
20


<210>  31
<211>  20
<212>  DNA
<213>  Artificial sequence

<220>
<223>  Primer


<220>
<221>  misc_feature
<222>  (1)..(20)
<223>  IL-12beta forward primer

<400>  31
gtccagctgg tcctttgttt
20


<210>  32
<211>  20
<212>  DNA

<213>  Artificial sequence

<220>
<223>  Primer


<220>
<221>  misc_feature
<222>  (1)..(20)
<223>  IL-12beta reverse primer

<400>  32
atcgttttgc tggtgtctcc
20


<210>  33
<211>  21
<212>  DNA
<213>  Artificial sequence

<220>
<223>  Primer


<220>
<221>  misc_feature
<222>  (1)..(21)
<223>  IL-1beta forward primer

<400>  33
tgtaatgaaa gacggcacac c
21


<210>  34
<211>  21
<212>  DNA
<213>  Artificial sequence

<220>
<223>  Primer


<220>
<221>  misc_feature
<222>  (1)..(21)
<223>  IL-1beta reverse primer

<400>  34
tcttctttgg gtattgcttg g
21

<210>  35
<211>  20
<212>  DNA
<213>  Artificial sequence

<220>
<223>  Primer


<220>
<221>  misc_feature
<222>  (1)..(20)
<223>  CXCL1 forward primer

<400>  35
agactccagc cacactccaa
20


<210>  36
<211>  18
<212>  DNA
<213>  Artificial sequence

<220>
<223>  Primer


<220>
<221>  misc_feature
<222>  (1)..(18)
<223>  CXCL1 reverse primer

<400>  36
tgacagcgca gctcattg
18


<210>  37
<211>  20
<212>  DNA
<213>  Artificial sequence

<220>
<223>  Primer


<220>
<221>  misc_feature
<222>  (1)..(20)
<223>  CCL20 forward primer

<400>  37

ggtactgctg gctcacctct
20


<210>  38
<211>  20
<212>  DNA
<213>  Artificial sequence

<220>
<223>  Primer


<220>
<221>  misc_feature
<222>  (1)..(20)
<223>  CCL20 reverse primer

<400>  38
tgtacgagag gcaacagtcg
20


<210>  39
<211>  19
<212>  DNA
<213>  Artificial sequence

<220>
<223>  Primer


<220>
<221>  misc_feature
<222>  (1)..(19)
<223>  BCL-2 forward primer

<400>  39
gtacctgaac cggcatctg
19


<210>  40
<211>  20
<212>  DNA
<213>  Artificial sequence

<220>
<223>  Primer


<220>
<221>  misc_feature
<222>  (1)..(20)

<223>  BCL-2 reverse primer

<400>  40
ggggccatat agttccacaa
20


<210>  41
<211>  21
<212>  DNA
<213>  Artificial sequence

<220>
<223>  Primer


<220>
<221>  misc_feature
<222>  (1)..(21)
<223>  CXCL2 forward primer

<400>  41
cctggttcag aaaatcatcc a
21


<210>  42
<211>  18
<212>  DNA
<213>  Artificial sequence

<220>
<223>  Primer


<220>
<221>  misc_feature
<222>  (1)..(18)
<223>  CXCL2 reverse primer

<400>  42
cttccgttga gggacagc
18

## Description

### Field of the Invention

The present invention relates to a method for the treatment or prevention of inflammatory disease or conditions, or inflammation as a symptom of a disease state. The invention also relates to a screening assay suitable for identifying agents capable of attenuating an inflammatory response in a mammal.

### Background to the Invention

Aberrant, uncontrolled or inappropriate inflammation underlies a wide range of human diseases including arthritis, multiple sclerosis and other autoimmune disorders, atherosclerosis, neurodegeneration and cancer. NF-κB is the master regulator of inflammation and controls the transcription of hundreds of genes that are critical for the inflammatory response. NF-κB is an inducible transcription factor. Under normal conditions, NF-κB is present in the cytoplasm of cells where it is unable to activate gene transcription. When a cell encounters an inflammatory stimulus, NF-κB translocates to the nucleus and it activates gene expression by binding to the promoter region of target genes. Reducing the levels of NF-κB transcriptional activity inhibits the inflammatory response and provides an effective therapy for human diseases where inflammation plays a role.

Ubiquitin Serine Protease 7 (USP7) (or herpesvirus-associated ubiquitin-specific protease (HAUSP)) is a ubiquitously expressed protein which proteolytically cleaves poly-ubiquitin chains from substrate proteins. Poly-ubiquitination of proteins is the major mechanism used in mammalian cells to induce the controlled proteasomal degradation of specific proteins. Poly-ubiquitination of substrate proteins is regulated by a complex set of proteins termed E1, E2 or E3 ubiquitin ligases. Once poly-ubiquitinated, a protein is recognised by the proteasome which catalyses its proteolytic degradation. USP7 opposes the function of the ubiquitin ligases by removing poly-ubiquitin chains and preventing proteasomal degradation of target proteins. Small-molecule inhibitors of USP7 have been described by Colland et al. (Small-molecule inhibitor of USP7/HAUSP ubiquitin protease stabilizes and activates p53 in cells". Mol. Cancer Ther. 2009; 8(8), pp: 2286-2295). Colland *et al.* describe a small-molecule compound which inhibits USP7's de-ubiquitinating activity towards p53, inhibiting cancer cell growth *in vitro.*

The current treatment for alleviating or preventing an inflammatory response is steroids, non-steroidal anti-inflammatory drugs (NSAIDs) and anti-Tumour Necrosis Factor-alpha (TNF-α) neutralising antibodies.

### Summary of the Invention

The Applicant has surprisingly discovered that USP7 specifically interacts with NF-κB and removes poly-ubiquitin chains. USP7 stabilises NF-κB and increases its transcriptional activity. Inhibition of USP7 activity decreases NF-κB transcriptional activity and reduces the expression of pro-inflammatory genes. Thus, USP7 inhibitors can prevent or alleviate inflammation, typically inflammation mediated by pro-inflammatory cytokines. The Applicant has further discovered that USP7 and NF-κB interact under conditions where NF-κB has translocated to the nucleus. Furthermore, it is demonstrated herein that USP7 interacts with NF-κB while bound to target gene promoters (for example, interleukin-6 (IL-6)). Thus USP7 is a gene-specific regulator of NF-κB which promotes NF-κB activity only at target genes. Inhibition of USP7 activity will likewise have a gene-specific effect on inhibiting NF-κB activity.

Accordingly, the invention broadly relates to a method for the treatment of inflammation in an individual, for example a causal therapy of a disease or condition for which an inflammatory state is an underlying cause of the disease, or a treatment of inflammation as a symptom of a disease state, for example inflammation caused by cancer, cardiovascular disease, an infection or trauma, by treating the individual with an agent capable of attenuating the activity of Ubiquitin Serine Protease 7 (USP7) protein (hereafter "USP7 inhibitor"), wherein the agent prevents the de-ubiquitination of NF-κB by USP7.

Suitably, the invention relates to a method for the symptomatic treatment of inflammation in an individual comprising a step of treating the individual with a USP7) inhibitor, wherein the USP7 inhibitor typically prevents the de-ubiquitination of NF-κB by USP7.

In a second aspect, the invention provides a ubiquitin serine protease 7 (USP7) inhibitor for use in the symptomatic treatment of inflammation in an individual, typically an individual having a disease or condition characterised by inflammation, wherein the USP7 inhibitor typically prevents the de-ubiquitination of NF-κB by USP7.

Suitably, the individual is a person having a disease or condition that involves inflammation, either acute or chronic (prolonged) inflammation. Thus, the method and use of the invention relates to the symptomatic prevention or alleviation of an inflammatory response in an individual having a disease or condition characterised by symptoms of inflammation. Thus, in one aspect, the invention relates to a symptomatic treatment, as opposed to a causal or prophylactic therapy. Examples of such diseases or conditions are provided below.

In another aspect, the invention relates to a method for the treatment of an inflammatory disease or condition in an individual comprising a step of treating the individual with an agent capable of attenuating the activity of Ubiquitin Serine Protease 7 (USP7) protein, wherein the agent typically prevents the de-ubiquitination of NF-κB by USP7.

Suitably, the disease or condition is a non-immune disease or condition, such as for example cancer, atherosclerosis or ischemic heart disease.

Typically, the disease or condition is an immune-mediated disease or condition, for example an inflammatory disorder or an autoimmune disease.

The USP7 inhibitor is typically selected from a USP7 ligand which directly inhibits USP activity (USP7 inhibitor ligand) and an agent capable of attenuating expression of USP7.

In one embodiment, the USP7 inhibitor ligand is selected from a USP7 antibody or antibody fragment, an inhibitory peptide, and a chemical inhibitor.

In one embodiment, the agent capable of attenuating expression of USP7 is selected from the group consisting of: siRNA, shRNA, miRNA, ribozyme, and antisense oligonucleotides.

The gene sequence of human USP7 is provided in GenBank Accession No: Z72499.1. Preferably, the agent capable of attenuating expression of USP7 is designed to target a section of human USP7 mRNA sense strand selected from: nucleotides 1160-1181; 1160-1179; nucleotides 961-978; and nucleotides 1003-1022. Ideally, the agent is a siRNA molecule.

The degenerative condition may be selected from the group consisting of arthritis, multiple sclerosis, atherosclerosis, neurodegeneration, and autoimmune diseases.

In another aspect of the present invention, there is provided a pharmaceutical composition for treating or preventing an inflammatory disease or condition in an individual, the composition comprising a ubiquitin serine protease 7 (USP7) inhibitor and a pharmaceutically acceptable carrier.

The USP7 inhibitor is typically selected from a USP ligand which directly inhibits USP7 activity (USP7 inhibitor ligand) and an agent capable of attenuating expression of USP7.

Suitably, the USP7 inhibitor ligand is selected from a USP7 antibody or antibody fragment, an inhibitory peptide, and a chemical inhibitor.

Typically the agent capable of attenuating expression of USP7 may be selected from the group consisting of: siRNA, shRNA, miRNA, ribozyme, and antisense oligonucleotides.

Generally, the inflammatory disease or condition may be selected from the group consisting of: a degenerative condition; a cardiovascular disease; and cancer, wherein the degenerative condition is selected from the group comprising inflammation-related diseases and disorders such as arthritis, multiple sclerosis, atherosclerosis, neurodegeneration, and autoimmune diseases.

The invention also relates to a USP7 inhibitor for use in preventing or alleviating an inflammatory response in an individual, typically an individual with an inflammatory disease or condition.

The invention also relates to a USP7 inhibitor for use in preventing or alleviating inflammation caused by a pro-inflammatory response in an individual, typically an individual with an inflammatory disease or condition.

The invention also relates to a method for the treatment of an inflammatory disease or condition in an individual comprising a step of administering a therapeutically effective amount of a USP7 inhibitor to the individual, with the proviso that treatment of cancer or viral-mediated diseases or conditions are excluded.

In a further aspect of the present invention, there is provided a method for identifying an agent capable of preventing or attenuation inflammation, or preventing or attenuating an inflammatory response, in an individual, which method employs a reaction mixture comprising USP7 and a target or substrate for USP7, adding a test agent to the reaction mixture and monitoring USP7 interaction with the USP7 target or substrate, wherein a decrease in USP7 interaction with the target or substrate relative to a reference reaction mixture that is free of the test agent indicates that the test agent is capable of attenuating an inflammatory response in the individual.

In a further aspect, the invention provides a method for identifying an agent capable of preventing or alleviating inflammation, or an inflammatory response, in an individual, which method employs a reaction mixture comprising USP7 and NF-κB, typically NF-κB expressing cells, adding a test agent to the reaction mixture and monitoring for de-ubiquitination of NF-κB, wherein a decrease in de-ubiquitination of NF-κB relative to a reference reaction mixture that is free of the test agent indicates that the test agent is capable of attenuating an inflammatory response in the individual.

In one embodiment, the reaction mixture comprises cells that express USP7 and NF-κB, and in which the method involves monitoring for interaction between USP7 and NF-κB.

In another embodiment, the reaction mixture may comprise a cell or cell-free system comprising a ubiquitin substrate, and in which the method involves monitoring for processing of the ubiquitin substrate.

### Brief Description of the Figures

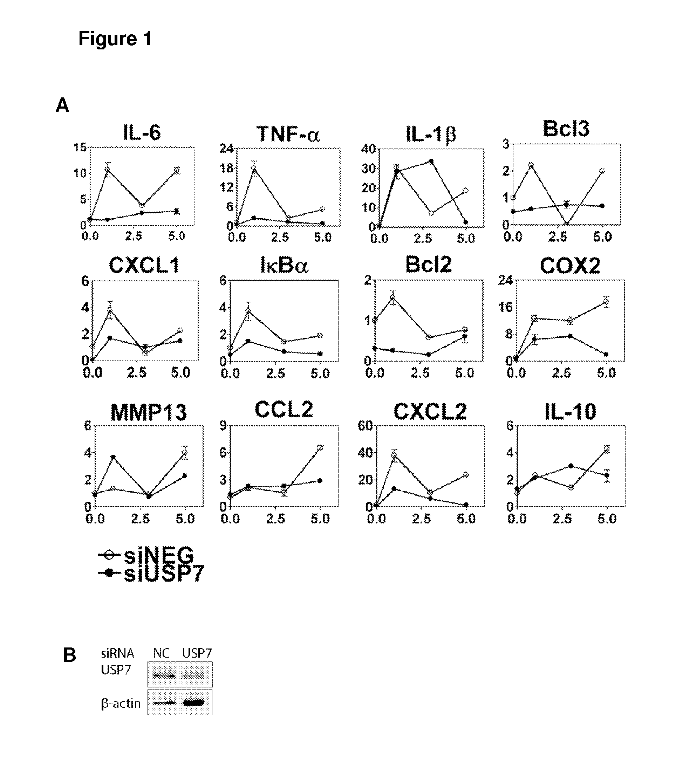

The invention will be more clearly understood from the following description of an embodiment thereof, given by way of example only, with reference to the accompanying drawings, in which:-
**Figure 1****. USP7 is required for LPS-induced gene expression.** (**A**) RAW 264.7 macrophage were transfected with control siRNA (siNEG) or siRNA directed against USP7 (siUSP7) using HiPerfect transfection reagent (Qiagen). 24hours post transfection cells were stimulated with 100 ng/ml lipopolysaccharide (LPS) for the indicated times before total RNA was extracted and used to quantitate gene expression levels by RealTime PCR. Data shown is mean ± SD of triplicate samples and representative of three independent experiments. (**B**) To verify USP7 knockdown RAW 264.7 macrophage transfected with control siRNA (NC) or siRNA directed against USP7 were lysed 24 hours post-transfection and immunoblotted with antibodies against USP7 and β-actin.

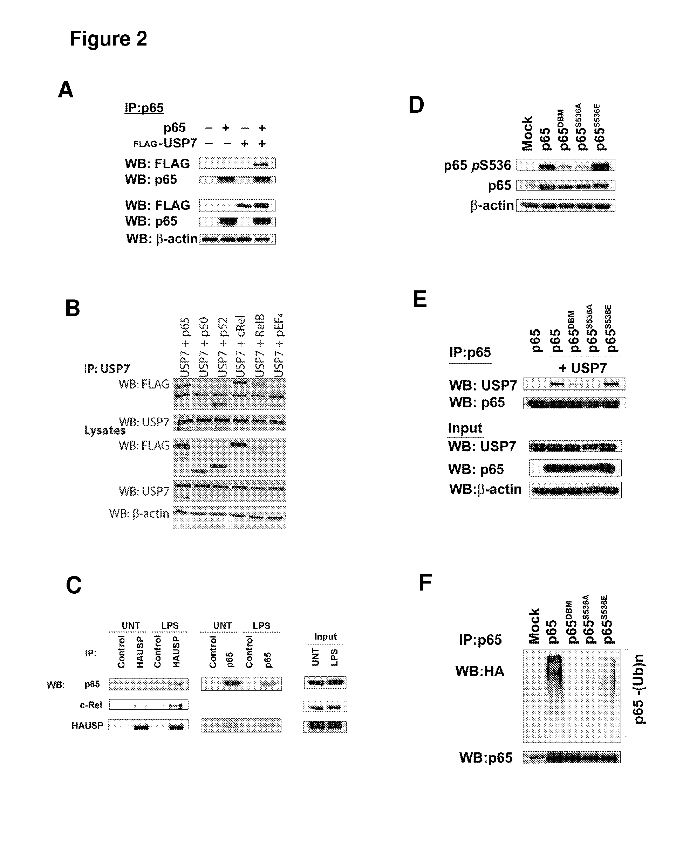

**Figure 2****. USP7 interacts with NF-κB subunits p65, p52, c-Rel, RelB but not p50.** (**A**) HEK293 cells were transfected with expression vectors for NF-κB p65 and FLAG-tagged USP7 as indicated using Turbofect transfection reagent (Fermentas). 24 hours post-transfection cells were lysed and p65 was immunoprecipitated using anti-p65 antibodies. Immunoprecipitates and lysates were analysed by immunoblot using anti-FLAG and anti-p65 antibodies. Immunoblotting with anti-β-actin antibody was used to verify equal protein loading. (**B**) HEK293 cells were transfected with expression vectors for NF-κB p65, p50, p52, c-Rel and RelB and USP7 as indicated using Turbofect transfection reagent (Fermentas). 24 hours post-transfection cells were lysed and USP7 was immunoprecipitated using anti-USP7 antibodies. Immunoprecipitates and lysates were analysed by immunoblot using anti-FLAG, anti-p65 and anti-xpress antibodies. Immunoblotting with anti-β-actin antibody was used to verify equal protein loading. (**C**) Interaction of endogenous USP7, p65 and c-Rel. RAW 264.7 macrophage were left untreated (UNT) or stimulated with lipopolysaccharide (LPS) for 60 minutes before preparation of whole cell lysates. USP7, p65 or c-Rel were immunoprecipitated using specific antibodies as indicated. Normal rabbit immunoglobulins (control) were used to demonstrate specificity of immunoprecipitation. Expression of p65, c-Rel and USP7 in RAW 264.7 macrophage lysates was confirmed by immunoblot. (**D-F**) Serine 536 and DNA binding of p65 is critical for its interaction with USP7. HEK293 cells were transfected with expression vectors for p65, p65 Y36A, E39D DNA binding mutant (DBM) p65 S536A, p65 S536E and FLAG-tagged USP7 as indicated using Turbofect transfection reagent (Fermentas). 24 hours post-transfection cells were lysed and p65 was immunoprecipitated using anti-p65 antibodies. Immunoprecipitates and lysates were analysed by immunoblot using anti-FLAG and anti-p65 antibodies. Immunoblotting with anti-β-actin antibody was used to verify equal protein loading.

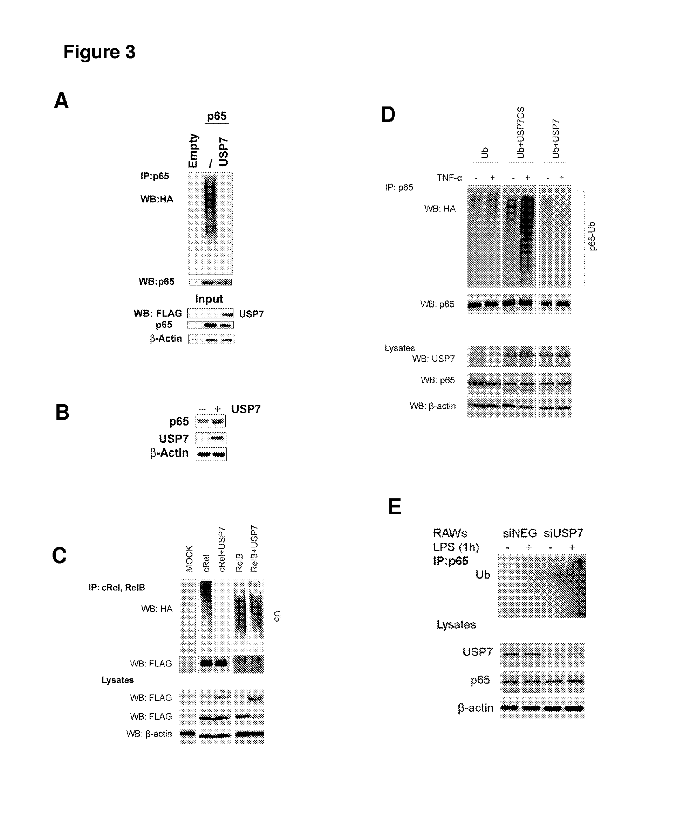

**Figure 3****. USP7 deubiquitinates NF-**κ**B**. (**A**) HEK293 cells were transfected with p65 expression plasmid and HA-tagged ubiquitin with or without FLAG-tagged USP7 vector. Total plasmid transfection was kept constant by the addition of the appropriate amount of empty expression vector. Cells transfected with empty expression vector and HA-tagged ubiquitin only were used as a control (Mock). 24 hours post-transfection cells were harvested and whole cell lysates prepared. Equal amounts of protein were then immunoprecipitated using anti-p65 antibody and immunoprecipitates resolved by SDS PAGE and immunoblotted using anti-HA antibody to detect poly-ubiquitinated p65. Lysates were immunoblotted with anti-p65, anti-FLAG and anti-β-actin to determine transfection efficiencies and protein loading. (**B**) USP7 expression increases p65 protein levels. HEK 293T cells were transfected with p65 and with or without FLAG-tagged USP7 expression plasmids. Protein expression levels were measured in whole-cell lysates by immunoblotting with anti-p65 and anti-FLAG antibodies using anti-β-actin antibodies as a control. (**C**) HEK293 cells were transfected with cRel and RelB expression plasmid and HA-tagged ubiquitin with or without FLAG-tagged USP7 vector. Total plasmid transfection was kept constant by the addition of the appropriate amount of empty expression vector. Cells transfected with empty expression vector and HA-tagged ubiquitin only were used as a control (Mock). 24 hours post-transfection cells were harvested and whole cell lysates prepared. Equal amounts of protein were then immunoprecipitated using anti-p65 antibody and immunoprecipitates resolved by SDS PAGE and immunoblotted using anti-HA antibody to detect poly-ubiquitinated cRel and RelB. Lysates were immunoblotted with anti-FLAG and anti-β-actin to determine transfection efficiencies and protein loading. (**D**) HEK293 cells were transfected with HA-tagged ubiquitin with or without FLAG-tagged USP7 ^{C223S} vector. Total plasmid transfection was kept constant by the addition of the appropriate amount of empty expression vector. Cells transfected with empty expression vector and HA-tagged ubiquitin only were used as a control (Ub). 24 hours post-transfection cells were harvested and whole cell lysates prepared. Equal amounts of protein were then immunoprecipitated using anti-p65 antibody and immunoprecipitates resolved by SDS PAGE and immunoblotted using anti-HA antibody to detect poly-ubiquitinated p65. Lysates were immunoblotted with anti-p65, anti-FLAG and anti-β-actin to determine transfection efficiencies and protein loading. (**E**) RAW 264.7 macrophage were transfected with control siRNA (siNEG) or siRNA directed against USP7 (siUSP7) using HiPerfect transfection reagent (Qiagen). 24hours post-transfection cells were stimulated with 100 ng/ml lipopolysaccharide (LPS) for 1 hour and whole cell lysates prepared. Lysates were immunoprecipitated using anti-p65 antibody and immunoprecipitates resolved by SDS PAGE and immunoblotted using anti-ubiquitin antibody to detect poly-ubiquitinated p65. Lysates were immunoblotted with anti-p65, anti-USP7and anti-β-actin to determine protein loading.

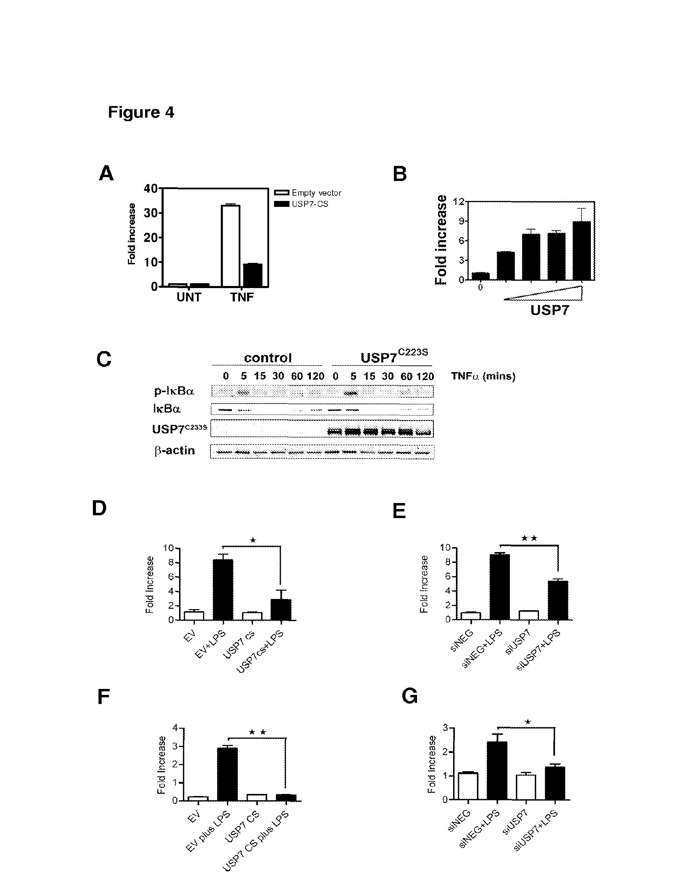

**Figure 4****. USP7 promotes NF-κB dependent gene expression.** (**A**) HEK293 cells were transfected with an NF-κB luciferase reporter plasmid along with empty expression vector or the dominant negative FLAG-tagged USP7 mutant C223S. The total amount of plasmid was kept constant using the empty vector as appropriate. Cotransfection of the Renilla luciferase expression vector pRLTK (Promega) was used as an internal control for the normalization of transfection across all samples. 24 hours post-transfection cells were left untreated (UNT) or treated with 20ng/ml TNF-α for 8 hours followed by a luciferase assay. Luciferase reporter activity is presented as fold increase over cells transfected with NF-κB luciferase reporter and empty expression vector. (**B**) HEK293 cells were transfected with an NF-κB luciferase reporter plasmid along with empty expression vector or increasing amounts of FLAG-tagged USP7 expression vector. The total amount of plasmid was kept constant using the empty vector as appropriate. Co-transfection of the Renilla luciferase expression vector pRLTK was used as an internal control for the normalization of transfection across all samples. Reporter activity is presented as fold increase over cells transfected with NF-κB luciferase reporter and empty expression vector. (**C**) HEK293 cells were transfected with the dominant negative FLAG-tagged USP7 mutant C223S and stimulated with 20ng/ml TNF-α for the indicated timepoints prior to lysis and immunoblotting with anti-IκBa, anti-phospho-IκBα anti-FLAG and anti-β-actin antibodies. (**D**) RAW 264.7 macrophage were transfected with an NF-κB luciferase reporter plasmid along with empty expression vector or the dominant negative FLAG-tagged USP7 mutant C223S. The total amount of plasmid was kept constant using the empty vector as appropriate. Co-transfection of the Renilla luciferase expression vector pRLTK (Promega) was used as an internal control for the normalization of transfection across all samples. 24 hours post-transfection cells were left untreated (UNT) or treated with 100ng/ml LPS for 8 hours followed by a luciferase assay. Luciferase reporter activity is presented as fold increase over cells transfected with NF-κB luciferase reporter and empty expression vector. (**E**) RAW 264.7 macrophage were transfected with an NF-κB luciferase reporter plasmid and constitutive Renilla luciferase reporter plasmid using Turbofect transfection reagent (fermentas). 24 hours later the cells were transfected with control siRNA (siNEG) or siRNA directed against USP7 (siUSP7) using HiPerfect transfection reagent (Qiagen). 24hours post transfection of siRNA cells were stimulated with 100 ng/ml lipopolysaccharide (LPS) for 8 hours and luciferase activity measured. Luciferase reporter activity is presented as fold increase over cells transfected with NF-κB luciferase reporter and control siRNA. (**F**) NIH 3T3 cells were transfected with an NF-κB luciferase reporter plasmid along with empty expression vector or the dominant negative FLAG-tagged USP7 mutant C223S. The total amount of plasmid was kept constant using the empty vector as appropriate. Co-transfection of the Renilla luciferase expression vector pRLTK (Promega) was used as an internal control for the normalization of transfection across all samples. 24 hours post-transfection cells were left untreated (UNT) or treated with 100ng/ml LPS for 8 hours followed by a luciferase assay. Luciferase reporter activity is presented as fold increase over cells transfected with NF-κB luciferase reporter and empty expression vector. (**G**) NIH 3T3 cells were were transfected with an NF-κB luciferase reporter plasmid and constitutive Renilla luciferase reporter plasmid using Turbofect transfection reagent (fermentas). 24 hours later the cells were transfected with control siRNA (siNEG) or siRNA directed against USP7 (siUSP7) using HiPerfect transfection reagent (Qiagen). 24 hours post-transfection of siRNA cells were stimulated with 100 ng/ml lipopolysaccharide (LPS) for 8 hours and luciferase activity measured. Luciferase reporter activity is presented as fold-increase over cells transfected with NF-κB luciferase reporter and control siRNA.

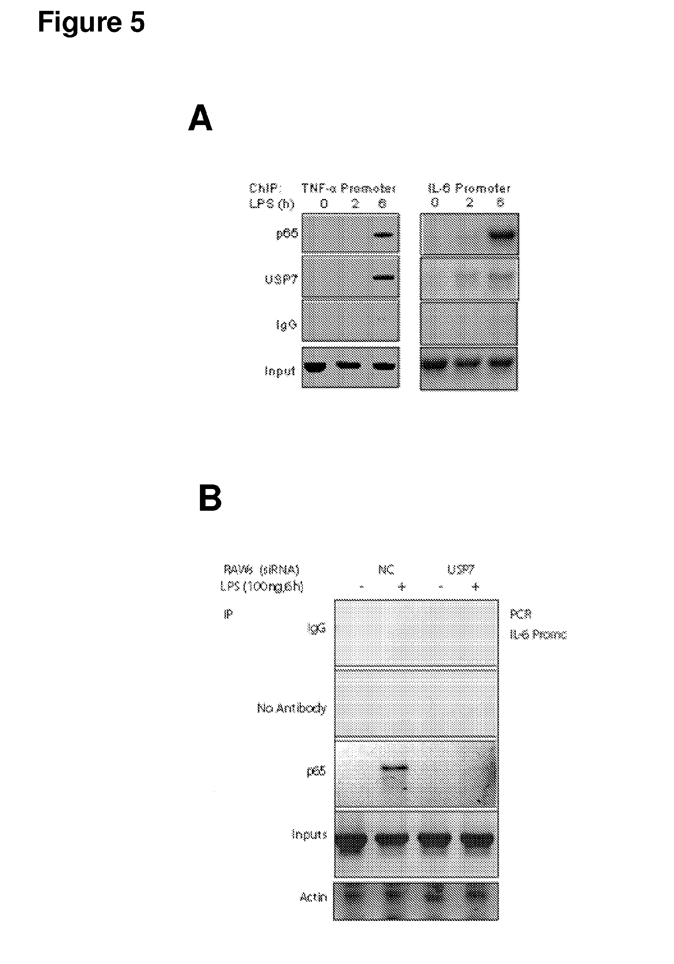

**Figure 5****. USP7 associates with NF-**κ**B target promoter.** (**A**) Bone marrow-derived macrophage were stimulated with 100ng/ml LPS for the indicated time periods following by chromatin immunoprecipitation using anti-p65, anti-USP7 and normal rabbit IgG antibodies. Chromatin immunoprecipitations were analysed using primers flanking the IL-6 promoter region. (**B**) RAW 264.7 macrophage were transfected with control siRNA (NC) or siRNA directed against USP7. 24 hours post-transfection cells were left untreated or treated with 100ng/ml LPS for 6 hours before chromatin immunoprecipitation was performed using the indicated antibodies. Chromatin immunoprecipitations were analysed using primers flanking the IL-6 promoter region and the β-atin promoter region.

### Detailed Description of the Invention

### Definitions

The term "symptomatic treatment of inflammation" should be understood to mean the prevention or alleviation of inflammation that occurs as a result of a pathological condition, be it chronic or acute inflammation. The term should in particular be understood to exclude causal or preventative treatments of diseases or pathologies; thus, in the context of an immune-mediated inflammatory disease such as rheumatoid arthritis, the treatment is aimed at preventing or alleviating inflammation as a symptom, as opposed to a causal or preventative treatment. Likewise, in the context of non-immune diseases such as cancer and cardiovascular disease, which is a disease which involves inflammation as a symptom of the cancer or cardiovascular disease, the treatment is directed to the prevention or alleviation of inflammation (as a symptom), as opposed to causal or preventative therapy of cancer or cardiovascular disease.

In this specification, the term "inflammatory disease or condition" should be understood to mean diseases or conditions that involve inflammation. This would include non-immune diseases with etiological origins in inflammatory processes, for example cancer and atherosclerosis, and immune mediated diseases or conditions which are primarily characterised by inflammation, for example inflammatory disorders and autoimmune diseases. Preferably, the term "inflammatory disease or condition" excludes cancer and diseases associated with viral infections.

In this specification, "autoimmune disease" should be understood to mean any disease which results in an aberrant immune response caused by a failure of the body to recognise self from non self. Typically, the autoimmune disease is selected from the group including but not limited to; Acute disseminated encephalomyelitis (ADEM); Addison's disease; Alopecia areata; Ankylosing spondylitis; Antiphospholipid antibody syndrome (APS); Autoimmune hemolytic anemia; Autoimmune hepatitis; Autoimmune inner ear disease; Bullous pemphigoid; Coeliac disease; Chagas disease; Chronic obstructive pulmonary disease; Crohns Disease; Dermatomyositis; Diabetes mellitus type 1; Endometriosis; Goodpasture's syndrome; Graves' disease; Guillain-Barré syndrome (GBS); Hashimoto's disease; Hidradenitis suppurativa; Kawasaki disease; IgA nephropathy; Idiopathic thrombocytopenic purpura; Interstitial cystitis; Lupus erythematosus; Mixed Connective Tissue Disease; Morphea; Multiple sclerosis (MS); Myasthenia gravis; Narcolepsy; Neuromyotonia; Pemphigus vulgaris; Pernicious anaemia; Psoriasis; Psoriatic Arthritis; Polymyositis; Primary biliary cirrhosis; Rheumatoid arthritis; Schizophrenia; Scleroderma; Sjögren's syndrome; Stiff person syndrome; Temporal arteritis (also known as "giant cell arteritis"); Ulcerative Colitis; Vasculitis; Vitiligo; Wegener's granulomatosis.

In this specification, the neurodegenerative disease is selected from the group consisting of: motor neurone disease (ALS) or variants thereof including primary lateral sclerosis and spinal muscular atrophy; prion disease; Huntington's disease; Parkinson's disease; Parkinson's plus; Tauopathies; Chromosome 17 dementias; Alzheimer's disease; Multiple sclerosis (MS); hereditary neuropathies; and diseases involving cerebellar degeneration.

In this specification, the term "cancer" should be taken to mean a cancer selected from the group consisting of: fibrosarcoma; myxosarcoma; liposarcoma; chondrosarcom; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; prostate cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias. In a preferred embodiment, the cancer is selected from the group consisting of: hematological, gastrointestinal, genitourinary, gynecological, thoracic head and neck, breast, and skin cancers.

In the context of the present invention, the term "cardiovascular disease" should be taken to mean a cardiovascular disease selected from the group comprising coronary heart disease, cardiomyopathy (for example, coronary artery disease, ischemic cardiomyopathy, hypertensive cardiomyopathy, valvular cardiomyopathy, inflammatory cardiomyopathy (such as endocraditis, inflammatory cardiomegaly, and myocarditis)), cardiovascular disease (for example, atherosclerosis), and ischemic heart disease.

"Treating" (or "treat") as used herein includes its generally accepted meaning which encompasses prohibiting, preventing, restraining, and slowing, stopping or reversing progression, severity, of a resultant symptom. As such, the methods of this invention encompass both therapeutic and prophylactic administration.

In this invention, the term "USP7 inhibitor" or "USP7 agent" should be understood to mean a molecule which suppresses the expression of USP7 protein (i.e. interferes with expression of the USP7 gene), including suppression of transcription or translation, and/or a molecule that directly inhibits USP7 activity, for example by binding to the USP7 protein (USP7 inhibitor ligand). Such inhibitors may comprise any of the group comprising siRNA (for example, as available from Santa Cruz Biotechnology Inc. (USA) (Catalogue Numbers sc-41521)), miRNA, shRNA (for example, see Santa Cruz Biotechnology Inc. (USA) Catalogue Number sc-41521-sh for shRNA plasmids effective for USP7), antisense oligonucleotides, ribozymes, peptides, ligands, chemical inhibitors, antibodies (for example, human USP7 primary antibodies are available from Abcam Inc. (USA) (Catalogue Numbers ab4080, ab84098, and ab71746) and Bethyl Laboratories Inc. (USA) (Catalogue Numbers A300-033A, A300-034A, A310-006A, IHC-00018)), and antibody fragments.

In one preferred embodiment of the invention, expression of USP7 is suppressed by means of RNA interference (RNAi). RNA interference (RNAi) is an evolutionally highly conserved process of post-transcriptional gene silencing (PTGS) by which double stranded RNA (known as siRNA molecules), when introduced into a cell, causes sequence-specific degradation of mRNA sequences. The RNAi machinery, once it finds a double-stranded RNA molecule, cuts it up, separates the two strands, and then proceeds to destroy RNA molecules that are complementary to one of those segments, or prevent their translation into proteins. Thus, suppression of USP7 expression may be achieved by treating an individual with siRNA molecules designed to target USP7 mRNA, preferably human USP7 and preferably a sequence in the human USP7 mRNA sense strand from nucleotides 1160 to 1181. More preferably, the siRNA molecules are designed to target a sequence in the human USP7 mRNA selected from the group comprising: nucleotides 1160 to 1179; nucleotides 961 to 978, and nucleotides 1003 to 1022. The siRNA molecule 5'- cccaaauuauuccgcggcaaa -3' sense strand (SEQUENCE ID NO: 1) specifically targets nucleotides 5'- cccaaattattccgcggca -3 at nucleotide positions 960-979 after the start codon of human USP7 (SEQ ID NO: 2). The siRNA molecule 5'-AAGCGUCCCUUUAGCAUUAUUUU-3' (SEQ ID NO: 3) specifically targets nucleotides 5'- AAGCGTCCCTTTAGCATTATT-3' sense strand (SEQ ID NO. 4) at nucleotide positions 761-778 after the start codon of human USP7.

siRNA oligonucleotides designed to target mouse USP7 genes are provided in SEQUENCE ID NO's: 6-9 below:
SEQ ID No 6: 5'-CAGGUAGACAUAGAUAAGGAA-3' (sense strand) specifically targets nucleotides 2937-2958 after the start site of mouse USP7;
SEQ ID No 7: 5'-AAAGAUCAUGAUGUAAUGUUA-3' (sense strand) specifically targets nucleotides 2043-2064 after the start site of mouse USP7;
SEQ ID No 8: 5'-CAGGAUUUAUCCAGGAUACUA-3' (sense strand) specifically targets nucleotides 2173-2194 after the start site of mouse USP7; and
SEQ ID No 9: 5'-CUGAUGGAUGGUGACAUCAUA-3' (sense strand) specifically targets nucleotides 2280-2301 after the start site of mouse USP7.

Other types of gene knockdown tools will be well known to the person skilled in the field of molecular biology. For example, micro RNA's (miRNAs) are small (~22nt) non-coding RNAs (ncRNAs) that regulate gene expression at the level of translation. Each miRNA apparently regulates multiple genes and hundreds of miRNA genes are predicted to be present in mammals. Recently miRNAs have been found to be critical for development, cell proliferation and cell development, apoptosis and fat metabolism, and cell differentiation. Alternatively, small hairpin RNA (shRNA) molecules are short RNA molecules having a small hairpin loop in their tertiary structure that may be employed to silence genes. The design of miRNA or shRNA molecules capable of silencing USP7 will be apparent to those skilled in the field of miRNA or shRNA molecule design. As an alternative, the level of USP7 expression can be modulated using antisense or ribozyme approaches to inhibit or prevent translation of USP7 mRNA transcripts or triple helix approaches to inhibit transcription of the USP7 gene. Antisense approaches involve the design of oligonucleotides (either DNA or RNA) that are complementary to USP7 mRNA. The antisense oligonucleotides will bind to the complementary mRNA transcripts and prevent translation.

Peptides (including variants and fragments thereof) of and for use in the invention may be generated wholly or partly by chemical synthesis or by expression from nucleic acid. The peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984)).

The term "chemical inhibitor" as employeded in the specification means a chemical compound that is capable of attenuating USP7 activity as determined using the USP7 activity assay provided below. For example, such assays would include a cellular de-ubiquitination assay as shown in Figure 3. In addition there are commercially available kits which are modified to include purified recombinant USP7, e.g. the DUB-Detector™ Deubiquitination Assay from Active Motif. This assay contains a fluorescent ubiquitin substrate that when processed by the cysteine protease class of DUBs (including USP7) releases a fluorescent signal that is proportional to the amount of enzymatic activity. Furthermore, screens may be performed by immunoprecipitation assays to measure the interaction of USP7 and NF-κB as shown in Figure 2.

The term "chemical inhibitor" within the meaning of the specification also includes those inhibitors of USP7 such as compounds on pages 5, 6, 7, and 8 of International Patent Publication No. WO 2010/081783, including their tautomers and their pharmaceutically acceptable salts (for example, as defined in Claim 10 of WO 2010/081783). Compounds having a 1,5-dihydro-pyrrol-2-one skeleton as defined in Claim 1 of WO 2010/081783 are also included. Compounds may be produced by the process as described in General Procedure 1, which results in Preparations A to F, as outlined on pages 23 to 27 of WO 2010/081783. These compounds are used as the basis for USP7 inhibitors as described in Examples 1 to 56 on pages 28 to 57 and the process of Claim 11 of WO 2010/081783. Other small molecule USP7 inhibitors include PR-619 (LifeSensors Inc., PA, USA), P5091 and P22077 (a structural analog of P5091) (Progenra Inc., PA, USA), including their tautomers and their pharmaceutically acceptable salts.

As used herein, the term "antibody" refers to an intact immunoglobulin or to a monoclonal or polyclonal antigen-binding fragment with the Fc (crystallizable fragment) region or FcRn binding fragment of the Fc region, referred to herein as the "Fc fragment" or "Fc domain", which is capable of specifically binding to USP7, preferably human USP7. Antigen-binding fragments may be produced by recombinant DNA techniques or by enzymatic or chemical cleavage of intact antibodies. Antigen-binding fragments include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the USP7 polypeptide. The Fc domain includes portions of two heavy chains contributing to two or three classes of the antibody. The Fc domain may be produced by recombinant DNA techniques or by enzymatic (e.g. papain cleavage) or via chemical cleavage of intact antibodies.

An immunoglobulin is typically a tetrameric molecule. As used herein, the term "immunoglobulin" refers to one or more chains of the tetrameric molecule. In a naturally occurring immunoglobulin, each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. In human, there are in addition four IgG (IgG1, IgG2, IgG3 and IgG4) and two IgA subtypes present. The variable regions of each light/heavy chain pair form the antibody binding site such that an intact natural immunoglobulin has two binding sites.

Immunoglobulin chains exhibit the same general structure: they include relatively conserved framework regions (FR) joined by three hypervariable regions, also called complementarity determining regions or CDRs. The CDRs from the two chains of each pair are aligned by the framework regions, enabling binding to a specific epitope. From N-terminus to C-terminus, both light and heavy chains comprise the domains FR1, CDR1, FR2, CDR2, FR3, CDR3 and FR4.

A "humanized antibody" is a genetically engineered antibody, wherein the mouse content is reduced to about 5-10%. In such cases, the six CDRs of the heavy and light chains and a limited number of structural amino acids of the murine monoclonal antibody are grafted by recombinant technology to the CDR-depleted human IgG scaffold. A fully human antibody or human antibody describes antibodies, which are made in humanized mice resulting in antibodies that do not contain any mouse sequences, or made in vitro using phage libraries or ribosome display or alternatively are obtained from human donors and which can bind specifically to USP7, especially human USP7. In certain embodiments, chimeric, humanized or primatized (CDR-grafted) antibodies, comprising portions derived from different species or fully human antibodies are also encompassed by the present invention. The various portions of these antibodies can be joined together chemically by conventional techniques, or can be prepared as a contiguous protein using genetic engineering techniques.

Various delivery systems are known and can be used to administer an inhibitor or composition of the invention, e.g., encapsulation in liposomes, microparticles, and microcapsules. Methods of introduction include but are not limited to intradermal, intramuscular, intraperitoneal, intravenous, subcutaneous, intranasal, epidural, and oral routes. The inhibitors or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the inhibitors or compositions of the invention into the circulation system by any suitable route. Pulmonary administration can also be employed, e.g., by use of an inhaler or nebulizer, and formulation with an aerosolizing agent.

It may be desirable to administer the inhibitors or compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

The present invention also provides pharmaceutical compositions. Such compositions comprise a therapeutically effective amount of a USP7 inhibitor (as defined above), and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals and more particularly in humans.

"Effective amount" refers to the amount or dose of the inhibitor, upon single or multiple dose administration to the patient, which provides the desired effect in the patient under treatment. An effective amount can be readily determined by the attending diagnostician, as one skilled in the art, by the use of known techniques and by observing results obtained under analogous circumstances. In determining the effective amount or dose of inhibitor administered, a number of factors are considered by the attending diagnostician, including, but not limited to: the species of mammal; its size, age, and general health; the specific disease involved; the degree of or involvement or the severity of the disease; the response of the individual patient; the particular inhibitor administered; the mode of administration; the bioavailabilty characteristics of the preparation administered; the dose regimen selected; the use of concomitant medication; and other relevant circumstances.

The term "carrier" refers to a diluent, adjuvant, excipient, or vehicle with which the compound or pro-drug of the invention is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol and water.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the USP7 inhibitor (compound or pro-drug of the invention), preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline.

In the case of cancer, the amount of the USP7 inhibitor (therapeutic of the invention) which will be effective in the treatment or prevention of cancer will depend on the type, stage and locus of the cancer, and, in cases where the subject does not have an established cancer, will depend on various other factors including the age, sex, weight, and clinical history of the subject. The amount of (therapeutic) may be determined by standard clinical techniques. In addition, *in vivo* and/or *in vitro* assays may optionally be employed to help predict optimal dosage ranges. The precise dose to be employed in the formulation will also depend on the route of administration, and the seriousness of the cancer, and should be decided according to the judgment of the practitioner and each patient's circumstances. Routes of administration of a therapeutic include, but are not limited to, intramuscularly, subcutaneously or intravenously. Effective doses may be extrapolated from dose-response curves derived from *in vitro* or animal model test systems. In the present invention, and without being bound by theory, it is believed that the cancer cells are affected by inhibition of NF-κB activity, the promoter of cancer cell survival, through inhibition of USP7 in cancer cells. Although the data suggests this model of inhibition, the inventors are not bound by this theory and acknowledge that other pathways promoting cancer cell survival may also be affected, leading to the death of cancer cells.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

### Brief Description of the Figures

### Materials and Methods

### Transfection of siRNA

1. Shortly before transfection, seed 0.4-2 x 10⁵ cells per well of a 24-well plate in 100 µl of an appropriate culture medium containing serum and antibiotics. Cells may alternatively be seeded after step 3 of this protocol.
2. For the short time until transfection, incubate the cells under normal growth conditions (typically 37°C and 5% CO2).
3. Dilute 375 ng siRNA in 100 µl culture medium without serum (this will give a final siRNA concentration of 50 nM after adding medium in step 7). Add 6 µl HiPerFect Transfection Reagent to the diluted siRNA and mix by vortexing. IMPORTANT: The amount of HiPerFect Transfection Reagent and siRNA required for optimal performance may vary, depending on the cell line and gene target.
4. Incubate the samples for 5-10 min at room temperature (15-25°C) to allow the formation of transfection complexes.
5. Add the complexes drop-wise onto the cells. Gently swirl the plate to ensure uniform distribution of the transfection complexes.
6. Incubate the cells with the transfection complexes under their normal growth conditions for 6 h.
7. Add 400 µl culture medium containing serum and antibiotics to the cells and incubate until analysis of gene silencing (e.g., 6-72 h after transfection, depending on the experimental setup). Change the medium as required. Note: The optimal incubation time for gene silencing analysis depends on the cell type, the gene targeted, and the method of analysis. This can be determined by performing a time-course experiment.

### Immunoprecipitation

**Day 1** Plate cells: 293T 2 x 10⁶cells/60mm dish 6ml complete media

**Day 2** *Transfect cells:* DNA 1:2 Turbofect, 100µl SFM/1µg DNA, Complex for 10mins. Added drop-wise to 100mm dish and incubated O/N at 37° C, 5% CO₂.

**Day 3** *Harvest cells:* Wash in 1ml ice-cold PBS, cells washed from plate with 1ml ice-cold PBS, remainder of cells washed with another ml of PBS. Centrifuge cells at 11,000 x g for 45 sec. Discard supernatant.

*Lyse cells:* Resuspend cells in 200µl RIPA Buffer (1ml RIPA Buffer, +10µl NaF (100mM), +10µl NaVO₃ (100mM), +10µl PMSF (100mM), 1µl Aprotenin (2500x), 1µl Pepstatin A (2500x), 1µl Leupeptin (2500x)). Incubate on ice for 30min, with vortexing every 5min. Centrifuge at max speed for 10min at 4° C, lysate (protein extract) is then quantified by Bradford Assay.

*Quantify:* Assay 1µl protein in 1ml of Assay Reagent (1X) against Standards of 0, 1.5, 3, 6, 7.5, 9µg/µl in 1ml of Assay Reagent.

Calculate the Lysates (30µg) and the highest concentration of IP possible with volume of lysate.

*Immunoprecipitation (IP):* 30µg Lysate samples were boiled with 2X Laemmli Buffer for 5min and stored at -20°C until IP's samples are ready. IP samples were brought to 1ml final volume in RIPA buffer. IP was precleared with incubation with agarose beads (20µl of 50% slurry) for 30min at 4° C on an end-to-end rotator. IP lysates were then placed in clean epp, 20µl agarose beads added and appropriate Antibody added. IP samples were incubated O/N at 4° C on an end-to-end rotator.

**Day 4** *Elute:* Centrifuge at 11,000 x g for 10sec at 4° C, wash 3 times with 1ml RIPA Buffer, invert 10 times and centrifuge at 11,000 x g for 10sec at 4° C. At the final wash, use a 23G needle attached to aspirator to remove all wash. Resuspend beads in 25µl 2X Laemmli Buffer and boil for 5mins. Vortex thoroughly and centrifuge at 16,000 x g for 2min at 4° C. Carefully remove 25µl of IP sample for clean eppie. Store samples at -20° C until processed further.

*Western Blot Analysis* Run 30µg lysate and 25µl IP samples in 10% gel at 100V for ~1h. Transfer to nitrocellulose membrane for 1h at 100V. Membrane blocked in 5% Blotto for 1 hour and incubated with appropriate antibody (in 5% Blotto) O/N at 4° C on rocker.

### Ubiquitination Assay

**Day 1** *Plate cells:* 293T 5 x 10⁶cells/100mm dish 10ml complete media

**Day 2** *Transfect cells:*
DNA 1:2 Turbofect, 100µl SFM/1µg DNA, Complex for 10mins.

Added drop-wise to 100mm dish and incubated O/N at 37° C, 5% CO₂.

**Day 3** *Harvest cells:* Wash in 1ml ice-cold PBS + 10mM NEM, wash cells from plate with 1ml ice-cold PBS + 10mM NEM, wash remainder of cells with another ml of PBS + 10mM NEM. Centrifuge cells at 11,000 x g for 45 sec. Discard supernatant.

*Lyse cells:* Resuspend cells in 100µl 1% SDS by flicking pellet. Vortex for 10s Boil for 5-7min and then sonicated for 3-6 sec to lose viscosity. Centrifuge at max speed for 10min at 4° C. Transfer lysate to clean eppie. Transfer 5µl to clean eppi and boil in 2X la Buffer (Lysates) for 5min, 1ml of RIPA+ (RIPA Buffer, +10µl NaF (100mM), +10µl NaVO₃(100mM), +10µl PMSF (100mM), 1µl Aprotenin (2500x), 1µl Pepstatin A (2500x), 1µl Leupeptin (2500x) and 20mM NEM) was added.

*IP:* samples were precleared with incubation with agarose beads (20µl of 50% slurry) for 30min at 4° C on an end-to-end rotator. Centrifuge at 14,000 x g for 2min. IP lysates were then placed in clean epp, 20µl agarose beads added and appropriate antibody added. IP samples were incubated O/N at 4° C on an end-to-end rotator.

**Day 4** *Elution:* Centrifuge at 11,000 x g for 10sec at 4° C, wash 3 times with 1ml RIPA plus 10mM NEM, invert 10 times and centrifuge at 11,000 x g for 10sec at 4° C. At the final wash, use a 23G needle attached to aspirator to remove all wash. Resuspend beads in 25µl 2X Laemmli Buffer and boil for 5mins. Vortex thoroughly and centrifuge at 16,000 x g for 2min at 4° C. Carefully remove 25µl of IP sample for clean eppie. Store samples at -20° C until processed further.

### Chromatin Immunoprecipitation (ChIP)

Active Motif ChIP-IT Express Kit with minor modification.

Optimised for: RAW246.7 Cells, Bone Marrow-derived Macrophage Cells, and 293T Cells.

### Cell Fixation

1. Grow cells in 10cm dish and treat cells as desired.
2. When cells are ready for harvest, pour media off the cells and add 10ml Fixation Soln (270µl of 37% formaldehyde to 10ml media) and incubate for 10min on a rocker.
3. Pour off fixation solution and wash with ice cold PBS for 5 sec.
4. Pour off wash and add 5ml of Glycine Stop-Fix Soln (0.5ml 10X Glycine buffer, 0.5ml 10X PBS and 4ml of dH2O) for 5min on a rocker.
5. Wash cells by pouring off Glycine Stop-Fix Soln, then add 5ml ice cold PBS for 5 sec.
6. Pour off and add 1ml of Cell Scraping soln (1ml PBS plus 5µl PMSF) and scrap cells. Transfer to epp.
7. Pellet cells at 2500rpm of 10min at 4°C.
8. Pellet can be used straight away or stored at -80°C with the addition of 1µl 100mM PMSF and 1µl of PIC.

### Shearing by sonication

1. Thaw pellet (if necessary) and resuspend in 0.5ml of ice cold Lysis buffer supplemented with 2.5µl PIC + 2.5µl PMSF. Incubate on ice for 30min.
2. Transfer the cells to an ice cold dounce homogenizer. Gently dounce on ice with 10 strokes to aid in nuclei release. Transfer cells to a 1.7ml epp and centrifuge at 5000rpm for 10min @4°C to pellet nuclei.
3. Carefully remove the supernatant and discard. Resuspend the nuclei pellet in 300µl Shearing buffer supplemented with 1.5µl PIC and place samples on ice.
4. Shear the DNA using optimised sonication conditions (6 pulses, 50% duty cycle and 30% output) Performed on ice with a 1min rest between each pulse to avoid overheating of the sample.
5. Centrifuge the sheared chromatin samples at 15,000rpm for 10min @4°C.
   Carefully transfer the supernatant to a fresh 1.7ml epp, this sheared chromatin can be used straight away or stored at -80°C.

### Assessing sonication

1. To assess sonication:
   a. To 50µl of shear chromatin add 150µl dH₂0 and 100µ 5M NaCl to each tube.
   b. Heat samples at 95°C in a water bath for 15min to reverse the cross links.
   c. Add 1µl RNaseA to each sample and incubate at 37°C for 15min
   d. Add 1µl Proteinase K to each sample and incubate at 67°C for 15min
   e. Column purify and spec to quantify DNA concentration
   f. Add 5µl of a 6X Loading buffer to 25µl of sample, load 5µl and 10µl of each sample in a 1% TBE agarose gel. Run at 100V for 45min-1h until loading dye reaches ¾ of the way to the end of the gel rack.
   g. Optimal sonciation shearing should result in a 200-1500bp smear.

### Column Purification (QIAquick Purification Kit)

Add 5 volumes of Buffer PB to 1 volume of the sample and mix, apply to column and apply vacuum. To wash, add 750µl of Buffer PE to each column and apply vacuum, transfer to 2ml collection tube. Remove residual ethanol from Buffer PE spin columns @ 13,000rpm for 1min. Discard collection tube and place columns in 1.5ml eppie. To elute, add 30-50µl of Buffer EB to the centre of the membrane in the column and centrifuge for 1min @ 13,000rpm. Store DNA @-20°C until further processed.

### Immunoprecipitation

1. Thaw chromatin (if necessary). Transfer 10µl to an epp (INPUT) which will be processed later.
2. Set up the ChIP reactions by adding the following components, note before
pipetting the magnetic beads, resuspend bead thoroughly.

| Reagent | 1 R^{xn} (<60µl of Chromatin) | 1 R^{xn} (>60µl of Chromatin) |
|---|---|---|
| Protein G Beads | 25µl | 25µl |
| ChIP Buffer 1 | 10µl | 20µl |
| Chromatin* | 20-60µl | 60-100µl |
| PIC | 1µl | 1µl |
| dH₂O | Upto 100µl R^{xn} volume | Upto 200µl R^{xn} volume |
| Antibody (added last) | 1-3µg | 1-3µg |
| Total Volume | 100µl | 200µl |

| | | |
|---|---|---|
| For p65 Santa Cruz sc372: 10µl For p50 Abcam ab-7971-1: 10µl For cRel Santa Cruz sc-71x: 1µl For USP7 Bertyl A300-033A: 1µl *Note: If the protocol is followed but not quantified, the chromatin (25µl) will contain ~ 4.µg of chromatin from a 10cm dish. Depending on the application, ChIP can be performed using anywhere from 1-50µg of chromatin. (For all reactions, 100µl of chromatin were used). | | |

1. Mix components and incubate on end-to-end rotator for 4h at 4°C. In some cases, sensitivity may be improved if the incubation is performed overnight.
2. Spin tubes to collect liquid from inside of the cap. Place tube on magnetic stand to pellet beads on the tube side. Carefully remove and discard supernatant.

### Wash Magnetic Beads

1. Wash beads one time with 800µl ChIP Buffer 1
2. Wash beads two times with 800µl ChIP Buffer 2
3. After the final wash, remove as much supernatant as possible without disturbing the beads. Use p200 if necessary.

### Elute Chromatin, Reverse Cross-links and Treat with Proteinase K

1. Resuspend washed beads with 50µl Elution Buffer AM2, incubate on end-to-end rotator for 15min
2. Spin to collect, add 50µl of Reverse cross-linking Buffer to eluted chromatin and immediately place in magnetic stand; allow beads to pellet.
3. Quickly transfer the supernatant to fresh eppie.
4. IPNUT DNA sample (10µl) was added to 88µl ChIP buffer 2 and 2µl 5M NaCl, so that its final volume is 100µl
5. Incubate the ChIP and INPUT DNA samples at 65°C for 2.5h
6. Return tubes to room temperature, spin to collect and then add 2µl of Proteinase K
7. Cap, mix and incubate at 37°C for 1h
8. Return tubes to room temperature, add 2µl Proteinase K Stop Solution. Briefly centrifuge to collect. DNA can be used straight away or stored at -20°C.

### PCR and qPCR

**1. PCR: Column purify the chromatin as outlined above.**

| Reagent | 1 R^{xn} |
|---|---|
| Template | 5µl |
| Primer (forward)(10µM) | 1µl |
| Primer (reverse)(10/µM) | 1µl |
| dNTPs | 1/µl |
| dH₂O | 14.3µl |
| 10X PCR Buffer | 2.5µl |
| Taq (5U/µl) | 0.2µl |
| Total Volume | 25µl |

| | |
|---|---|
| 1. Dilute the INPUT 1/10 prior to application and include IgG and water controls. 2. Programme the thermocycler to initial melt step at 94°C for 3min, then 36 cycles of 94°C for 20sec, 59°C for 30sec and 72°C for 30sec; then hold cycle at 8°C. 3. Run ~8µl of each PCR product on a 3% agarose gel | |

**4. qPCR: Column purify the chromatin as outlined above.**

| Reagent | 1 R^{xn} | 3.5 R^{xn} |
|---|---|---|
| Template | 2µl | 7µl |
| Primer (forward)(10µl) | 0.2µl | 0.7µl |
| Primer (reverse)(10/µM) | 0.2µl | 0.7µl |
| SYBR | 5µl | 17.5µl |
| dH₂O | 2µl | 7µl |
| Total Volume | 10µl | 35µl |

| | | |
|---|---|---|
| 1. 10µl of sample was loaded in triplicate, cover with sticky lid and spin to collect liquid at the bottom of the tube. 2. Run on qPCR machine (AQ programme). Programme the thermocycler to initial melt step at 94°C for 3min, then 36 cycles of 94°C for 20sec, 59°C for 30sec and 72°C for 30sec; then hold cycle at 8°C, data collected after each cycle. 3. Run 10µl of each PCR product on a 3% agarose gel. | | |

### Luciferase assay

Wash cells in phosphate buffer saline. Add 100µl of Passive Lysis Buffer (Promega) per 24 will plate. Incubate with gentle rock at room temperature for 15 minutes.

**I. Firefly Luciferase Assay Buffer**

| | **STOCK** | **FINAL** | **vol/5mls** |
|---|---|---|---|
| **Glycylglycine** | 250mM | 25mM | 500 ul |
| **K₂PO₄ pH8** | 1M | 15mM | 75u l |
| **EGTA** | 200mM | 4mM | 100u l |
| **MgSO₄** | 500mM | 15mM | 150 ul |
| **CoA** | 10mM | 0.1mM | 50ul |
| **Luciferin** | 7.5mM | 75 M | 50 ul |
| **DTT** | 500mM | 1mM | 10u l |
| **ATP** | 200mM | 2mM | 50 ul |
| **H₂O** | | | 3.915ml |

**IL Renilla Luciferase Assay Buffer**

| | **STOCK** | **FINAL** | **vol/5mls** |
|---|---|---|---|
| **NaCl** | 2.5M | 1.1 M | 2.2ml |
| **EDTA** | 500mM | 2.2mM | 22 ul |
| **K₂PO₄ pH5.1** | 1M | 220mM | 1.111ml |
| **BSA** | 100mg/ml | 0.44mg/ml | 22u l |
| **NaN₃** | 1M | 1.3mM | 6.5u l |
| **Coelentrerazine*** | 1.43mM | 1.43 M | 5 ul* |
| **H₂O** | | | 1.633ml |

| | | | |
|---|---|---|---|
| * Add just before use | | | |

### Protocol

Add 5-10 µl lysate to 50 µl Firefly Luciferase Assay buffer, mix by pipetting. Read sample. Add 50 µl Renilla Luciferase Assay buffer, mix by pipetting. Read sample.

### List of primers used:

### ChIP Primers

| **Primer** | **Direction** | **Sequence (5'-3')** | **Reference** |
|---|---|---|---|
| IL-6 | Forward | CGTTTATGATTCTTTCGATGCTAAACG (SEQ ID NO: 12) | Reddy et al., 2008 |
| | Reverse | GTGGGGTCCAGAGCAGAATGAG (SEQ ID NO: 13) | 205bp |
| TNF-α | Forward | GAGGCTCCGTGGAAAACTCACTTG (SEQ ID NO: 14) | |
| | Reverse | GCAGAGCAGCTTGAGAGTTGGGAA (SEQ ID NO: 15) | |
| IκBα | Forward | TCAAATCGATCGTGGGAAA (SEQ ID NO: 16) | Ashall et al., 2009 |
| | Reverse | GGACTGCTGTGGGCTCTG (SEQ ID NO: 17) | 360bp |
| β-actin | Forward | TCGATATCCACGTGACATCCA (SEQ ID NO: 18) | 269bp |
| | Reverse | GCAGCATTTTTTTACCCCCTC (SEQ ID NO: 19) | Maeda et al., Nature |

### qPCR Probe Library Primers

| **Primer** | **Nucleotide Accession no.** | **Direction** | **Sequence (5'-3')** | **Probe** |
|---|---|---|---|---|
| IL-6 | BC138766 | Forward | TGATGGATGCTACCAAACTGG | 6 |
| | | Reverse | TTCATGTACTCCAGGTAGCTATGG | 6 |
| TNF-α | AK153319.1 | Forward | TCTTCTCATTCCTGCTTGTGG | 49 |
| | | Reverse | GGTCTGGGCCATAGAACTGA | 49 |
| IκBα | AK089215.1 | Forward | ACGAGCAAATGGTGAAGGAG | 38 |
| | | Reverse | ATGATTGCCAAGTGCAGGA | 38 |
| 18S rRNA | BK000964.1 | Forward | AAATCAGTTATGGTTCCTTTGGTC | 55 |
| | | Reverse | GCTCTAGAATTACCACAGTTATCCAA | 55 |
| IL-10 | AK152344.1 | Forward | CAGAGCCACATGCTCCTAGA | 41 |
| | | Reverse | GTCCAGCTGGTCCTTTGTTT | 41 |
| IL-12β | AF128214.1 | Forward | GTCCAGCTGGTCCTTTGTTT | 78 |
| | | Reverse | ATCGTTTTGCTGGTGTCTCC | 78 |
| IL-1β | AK156396.1 | Forward | TGTAATGAAAGACGGCACACC | 78 |
| | | Reverse | TCTTCTTTGGGTATTGCTTGG | 78 |
| CXCL1 | AK140312.1 | Forward | AGACTCCAGCCACACTCCAA | 83 |
| | | Reverse | TGACAGCGCAGCTCATTG | 83 |
| CCL20 | AB015136.1 | Forward | GGTACTGCTGGCTCACCTCT | 29 |
| | | Reverse | TGTACGAGAGGCAACAGTCG | 29 |
| BCL-2 | AA867214.1 | Forward | GTACCTGAACCGGCATCTG | 75 |
| | | Reverse | GGGGCCATATAGTTCCACAA | 75 |
| CXCL2 | AK137628.1 | Forward | CCTGGTTCAGAAAATCATCCA | 63 |
| | | Reverse | CTTCCGTTGAGGGACAGC | 63 |

### siRNA Sequences

| **Target** | **USP7** (Mouse) |
|---|---|
| **Target Sequence** | 5'- CAGGTAGACATAGATAAGGAA -3' SEQ ID NO: 9 |
| **Sense strand** | 5'- GGUAGACAUAGAUAAGGAATT -3' SEQ ID NO: 10 |
| **Antisense strand** | 5'- UUCCUUAUCUAUGUCUACCTG -3' SEQ ID NO: 11 |
| | |

| **Target** | **USP7** (Human) |
|---|---|
| **Target Sequence** | 5'- CCCAAAUUAUUCCGCGGCAAA -3' SEQ ID NO: 2 |
| **Sense strand** | 5'- CCCAAAUUAUUCCGCGGCAAA -3' SEQ ID NO: 3 |
| **Antisense strand** | 5'- UUUGCCGCGGAAUAAUUUGGG -3' SEQ ID NO: 5 |

### Allstars Negative Control siRNA (Qiagen, Cat. No. 1027280)

### Results

### USP7 is required for LPS-induced gene expression.

The requirement of USP7 for LPS-induced gene expression in macrophage was demonstrated by transfection of RAW264.7 macrophage cells with siRNA directed against USP7 or a negative control siRNA. Efficiency of USP7 knockdown was established by immunoblotting of lysates from transfected cells with anti-USP7 antibody. As illustrated in Figure 1B, approximately 80% reduction in USP7 protein in cells transfected with siRNA for USP7 relative to control cells was observed. 24hours post-transfection cells were treated with 100ng/ml LPS and cells harvested for total RNA extraction after 1, 3 and 5 hours. The relative levels of mRNA of pro-inflammatory cytokines and chemokines (CCL2, IL-1β, TNF-α, IL6, IL10, CXCL2, CXCL1, MMP13) and other target genes of NF-κB (IκBα, BCL2, BCL3, COX2) were analysed by real-time PCR (Figure 1A). This analysis revealed a significant reduction in the expression of LPS-induced genes in USP7 knockdown cells demonstrating a critical role for USP7 in the regulation of the LPS/Toll-like receptor 4-induced inflammatory response.

### USP7 promotes NF-κB transcriptional activity.

The transcription factor NF-κB is the master regulator of the inflammatory response and regulates the expression of hundreds of genes in response to a range of inflammatory stimuli (www.nf-kb.org). As knockdown of USP7 significantly inhibited the LPS-induced expression of a number of NF-κB regulated genes we next determined the role of USP7 in NF-κB mediated gene transcription. Initially a NF-κB luciferase reporter assay was performed in HEK293T cells transfected with a dominant negative mutant of USP7 in which the critical cysteine residue in the active site is mutated to a serine (USP7^{C221S}). This mutant does not possess proteolytic activity and serves as a dominant negative mutant. As illustrated in Figure 4A, expression of USP7^{C223S} significantly inhibits (three-fold reduction) NF-κB reporter activity in cells treated with TNFα.

In contrast to the effect of loss of USP7 function on NF-κB activity, Figure 4B illustrates a dose-dependent increase in NF-κB reporter activity when HEK293T cells were transfected with increasing amounts of USP7 expression vector, further supporting a role for USP7 in promoting NF-κB transcriptional activity.

To rule out the possibility that USP7^{C223S} inhibits the activation of NF-κB proximal to the TNFα receptor, the phosphorylation and degradation of IκBα following TNFα stimulation of USP7^{C223S} expressing cells were examined. As Figure 4B illustrates, USP7^{C223S} does not inhibit the phosphorylation and degradation of IκBα following TNFα stimulation, indicating that the inhibition of NF-κB activity by USP7^{C223S} does not occur at the activation stage. Interestingly in cells expressing USP7 ^{C223S} the NF-κB-dependent synthesis of IκBα is reduced following TNFα stimulation which correlates with the reduced expression of IκBα observed in USP7 siRNA transfected RAW macrophage stimulated with LPS (see Figure 1A).

NF-κB reporter activity in LPS treated RAW macrophage transfected with USP7^{C223S} and USP7 siRNA are illustrated in Figure 4D and Figure 4E, respectively. In both cases a significant reduction in NF-κB reporter activity was observed following LPS stimulation. Similar data was obtained using NIH3T3 fibroblasts demonstrating that USP7 is a broad regulator of NF-κB activity as illustrated in Figure 4F and 4G.

### USP7 interacts with NF-κB.

The ubiquitination of NF-κB has been demonstrated to be a major regulatory mechanism controlling its transcriptional activity suggesting that NF-κB may be a novel substrate for the de-ubiquitinase activity of USP7. Firstly, the ability of USP7 to interact with NF-κB was investigated. Initial experiments were carried out by transfecting HEK293T cells with expression vectors for FLAG tagged-USP7 and the NF-κB subunit RelA/p65. Following immunoprecipitation of lysates with anti-RelA/p65 antibodies, interaction with USP7 was assessed by immunoblot using anti-FLAG antibody. Figure 2A illustrates USP7 was readily detectable in anti-RelA/p65 immunoprecipitates from co-transfected cells, demonstrating that USP7 and NF-κB are capable of interacting. Similar experiments were performed using expression vectors for the NF-κB subunits p50, p52, c-Rel and RelB. As Figure 2C shows, USP7 co-immunoprecipitated with all subunits of NF-κB except p50, indicating that USP7 targets multiple NF-κB complexes.

The interaction of the endogenous USP7 and NF-κB proteins in RAW macrophage was investigated. As Figure 2C shows, very low levels of p65 were detected in anti-USP7 immunoprecipitates in unstimulated cells. However, following LPS stimulation, a significant level of RelA/p65 co-immunoprecipitated with USP7 demonstrating an inducible interaction between NF-κB and USP7. Similar results were observed with cRel. The inducibility of this interaction is most readily explained by the subcellular localisation of both proteins in untreated and LPS-stimulated cells. USP7 is a predominantly nuclear protein while NF-κB is predominantly cytosolic in unstimulated cells.

Nuclear localisation of NF-κB occurs following activation and so the interaction of USP7 and NF-κB is inducible. DNA binding and phosphorylation of NF-κB have previously been demonstrated as triggers for NF-κB ubiquitination. To determine if these events also regulate the interaction of USP7 and NF-κB,mutants of RelA/p65 which are defective in DNA binding (Y36A, E39D) and phosphorylation at serine 536 (S536A) were utilised. Co-immunoprecipitation experiments revealed that DNA binding and phosphorylation at serine 536 regulate the interaction of RelA/p65 and USP7. As illustrated in Figures 2D and 2E, both the DNA binding mutant and the S536A mutant of RelA/p65 demonstrated significantly reduced interaction with USP7 with the S536A mutation, virtually abolishing USP7 interaction. This indicates that the events which trigger the ubiquitination of NF-κB is intimately linked to those that regulate NF-κB de-ubiquitination.

### USP7 de-ubiquitinates NF-κB

In order to determine if NF-κB is a substrate for the de-ubiquitinase activity of USP7, an *in vitro* ubiquitination assay was performed. HEK293T cells were transfected with RelA/p65 and HA-tagged ubiquitin in the presence and absence of FLAG-tagged USP7. Following denaturing lysis and anti-RelA/p65 antibody, immunoprecipitates were immunoblotted using anti-HA antibody. As Figure 3A illustrates, co-expression of USP7 significantly reduced the ubiquitination of RelA/p65. This data demonstrates that USP7 is a de-ubiquitinase of NF-κB. The polyubiquitination of RelA/p65 leads to its proteasomal mediated degradation thus de-ubiquitination should lead to an accumulation of protein. Indeed immunoblot analysis revealed increased levels of RelA/p65 protein when USP7 is co-expressed, as illustrated in Figure 3B.

In order to assess the effect of USP7 knockdown on NF-κB ubiquitination, RAW cells were transfected with USP7 siRNA and the endogenous ubiquitination of RelA/p65 following LPS stimulation was measured. As illustrated in Figure 3E, knock down of USP7 increased the ubiquitination of RelA/p65 following LPS stimulation indicating that the reduced LPS-induced gene expression seen in RAW cells following USP7 knockdown is due to increased NF-κB ubiquitination and degradation. Similarly, HEK293T cells expressing the dominant negative USP7^{C223S} display increased p65 ubiquitination following TNFα stimulation (Figure 3D). Together these data demonstrate that USP7 regulates the ubiquitination of NF-κB.

### USP7 associates with NF-κB target promoters.

The binding of NF-κB to DNA is a critical event in triggering its ubiquitination. Indeed, RelA/p65 mutants which are resistant to signal -induced degradation remain bound to target promoters for much longer periods than wild type RelA/p65. This indicates that the ubiquitination of NF-κB may occur while it is DNA bound and suggests that USP7 mediated de-ubiquitination may also occur on DNA bound NF-κB. In order to explore this possibility, chromatin immunoprecipitation experiments with LPS-stimulated RAW cells using antibodies specific for RelA/p65 and USP7 were performed. As illustrated in Figure 5A, LPS stimulation induced the concomitant association of RelA/p65 and USP7 with the promoters of both TNFα and IL-6. Moreover, siRNA knockdown of USP7 resulted in a loss of RelA/p65 binding to the IL-6 promoter following LPS stimulation (Figure 5B). These data demonstrate that USP7 associates with the promoter regions of NF-κB target genes promoting the binding of NF-κB at those sites. They also demonstrate that USP7 is important for the occupancy of promoters by NF-κB and subsequent transcriptional activity.

The invention is not limited to the embodiments hereinbefore described but may be varied in both construction and detail.

## Claims

1. A ubiquitin serine protease 7 (USP7) inhibitor for use in the symptomatic treatment of inflammation in an individual, wherein the USP7 inhibitor prevents the de-ubiquitination of NF-κB by USP7.

2. A use according to Claim 1 in which the individual has an inflammatory disease or condition.

3. A use as claimed in Claim 2 in which the inflammatory disease or condition is an immune-mediated disease or condition.

4. A use as claimed in Claim 3 in which immune-mediated disease or condition is an inflammatory disorder.

5. A use as claimed in Claim 3 in which the immune-mediated disease or condition is an autoimmune disease.

6. A use as claimed in Claim 2 in which the inflammatory disease or condition is a non-immune disease with etiological origins in inflammatory processes, and selected from cancer, cardiovascular disease and ischemic disease.

7. A use as claimed in any of Claims 1 to 5, in which the inflammatory disease or condition is selected from the group consisting of a degenerative condition, a cardiovascular disease, and cancer, and wherein the USP7 inhibitor is employed for the symptomatic prevention or alleviation of an inflammatory response.

8. A use as claimed in Claim 7, in which the degenerative condition is selected from the group consisting of arthritis, multiple sclerosis, atherosclerosis, neurodegeneration, and autoimmune diseases, and wherein the USP7 inhibitor is employed to for the symptomatic prevention or alleviation of an inflammatory response.

9. A use as claimed in any preceding Claim in which the USP7 inhibitor is a chemical having a 1,5-dihydro-pyrrol-2-one skeleton.

10. A method for identifying an agent capable of preventing or alleviating an inflammatory response in an individual, which method employs a reaction mixture comprising USP7 and NF-κB, adding a test agent to the reaction mixture and monitoring for de-ubiquitination of NF-κB, wherein a decrease in de-ubiquitination of NF-κB relative to a reference reaction mixture that is free of the test agent indicates that the test agent is capable of attenuating an inflammatory response in the individual.

11. A method as claimed in Claim 10 in which the reaction mixture comprises cells that
express USP7 and NF-κB.

12. An in-vitro method of inhibiting de-ubiquitination of NF-κB comprising a step of reacting a sample of NF-κB cells with a USP7 inhibitor.

13. A USP7 inhibitor for use in the treatment or prevention of an inflammatory disease
or condition in an individual by preventing de-ubiquitination of NF-κB by USP7.

14. Use as claimed in Claim 13 in which the USP7 inhibitor is a chemical having a 1,5-dihydro-pyrrol-2-one skeleton.
